# EUROPÄISCHE PATENTSCHRIFT

(11) **EP 1 731 906 B1**
(45) Veröffentlichungstag und Bekanntmachung des Hinweises auf die Patenterteilung: **26.03.2008**
(21) Anmeldenummer: 06011924.5
(22) Anmeldetag: 09.06.2006
(51) Int. Cl.: G01N 33/38

(54) **Vorrichtung zur Bestimmung der korrespondierenden Luftfeuchtigkeit einer Materialprobe und zur Bestimmung des Feuchtigkeitsgehaltes dieser gleichen Materialprobe in einem geschlossenen System**
Device for determining the corresponding air humidity of a material sample and for determining the moisture content of this same material sample in a closed system
Dispositif destiné à la détermination de l'humidité de l'air correspondant d'un échantillon de matériau et à la détermination de la teneur en humidité de ce même échantillon de matériau dans un système fermé

(30) Priorität: 10.06.2005 CH 9882005
(43) Veröffentlichungstag der Anmeldung: 13.12.2006
(73) Patentinhaber: Dr. Radtke CPM Chemisch-Physikalische Messtechnik AG, 6340 Baar (CH)
(72) Erfinder: Radtke, Frank, 6300 Zug (CH)
(74) Vertreter: Zink-Wild, Markus Peter

(56) Entgegenhaltungen:
- DD-A1- 273 685
- DE-A1- 10 203 637
- US-A- 4 441 244
- US-A- 6 073 480

## Beschreibung

Die vorliegende Erfindung betrifft eine Vorrichtung zur Bestimmung der korrespondierenden Luftfeuchtigkeit einer Materialprobe und zur Bestimmung des Feuchtigkeitsgehaltes dieser gleichen Materialprobe in einem geschlossenen System.

Frisch eingebrachte Unterlagsböden, auch Estrich oder "screed (E)" genannt, müssen bis zur Belegereife unter anderem einen wesentlichen Teil ihrer Feuchtigkeit durch Abbinden und/oder durch Verdunsten abgegeben haben. Wird ein Unterlagsboden belegt, bevor er genügend trocken ist, so führt dies mit hoher Wahrscheinlichkeit zur Schädigung des Oberbelages: Bei einem Parkettboden lösen sich die Riemen ab, bei einem dampfdichten Belag bilden sich Blasen, bei einem Fliesen- oder Steinbelag platzen die Fliesen resp. die Steinplatten ab.

Es liegt in der Verantwortung eines jeden Handwerkers, beim Prüfen der Belegereife eine brauchbare und hinreichend genaue Feuchtigkeitsmessung durchzuführen, wie dies beispielsweise in der Schweizerischen Norm SIA 253, in der Deutschen Norm DIN 18365 oder in der Italienischen Norm UNI 10329 vorgeschrieben ist.

In der Schweiz und in Deutschland wird zur Messung der in einem Unterlagsboden - es kann auch eine Betondecke sein - enthaltenen Feuchtigkeit die so genannte Calciumcarbid-Methode (CM-Methode) angewendet.

Geeignete Vorrichtungen werden in den Dokumenten DE 102005002248 (nach dem Prioritätstag veröffentlicht) und DD273685 beschrieben.

Dabei wird dem Unterlagsboden eine Materialprobe entnommen und zusammen mit Calciumcarbid, welches in eine Ampulle aus Glas abgepackt ist, in eine Druckflasche gegeben. In diese Druckflasche werden als Misch- und Mahlelemente zusätzlich Stahlkugeln oder andere Gegenstände, beispielsweise Muttern, gegeben. Üblicherweise werden 4 Stahlkugeln hinzugegeben. Anschliessend wird die Druckflasche verschlossen. Die Messung wird durch manuelles, intensives und senkrechtes Schütteln der Druckflasche gestartet. Dabei bewirken die Stahlkugeln, dass einerseits die Glasampulle mit dem Calciumcarbid zerbrochen wird und andererseits das Probenmaterial und das freigesetzte Calciumcarbid vermischt und zerkleinert werden. Das im Probenmaterial enthaltene Wasser reagiert mit dem Calciumcarbid und bildet unter anderem ein Gas, nämlich Acetylen. Der entstehende Druck wird mit einem Druckmessgerät gemessen und bildet die Messgrösse zur Bestimmung des Feuchtigkeitsgehaltes der Materialprobe.

Mit dieser Messmethode wird die Feuchtigkeit einer Materialprobe in einem geschlossenen System innerhalb von wenigen Minuten bestimmt.

In den skandinavischen Ländern sowie in England wird ein anderes einfaches Messverfahren zur Bestimmung der Belegereife eingesetzt. Dieses Messverfahren wird in den Englischen Normen BS5325, BS8201 und BS8203 vorgeschrieben. Die Belegereife wird mit einer Luftfeuchte-/Lufttemperaturmessvorrichtung festgestellt. Dazu bohrt man in den zu untersuchenden Boden, beispielsweise ein Unterlagsboden oder eine Betondecke, ein Loch in hinreichender Tiefe und misst dort nach einer gewissen Wartezeit, in der Regel etwa 5 bis 7 Tagen, die Luftfeuchtigkeit.

Mit dieser Messmethode wird im Bohrloch der Gleichgewichtszustand der Feuchtigkeit zwischen dem Unterlagsboden oder der Betondecke und der Luftfeuchtigkeit im Bohrloch bestimmt. Bei dieser Messmethode handelt es sich um eine Messung in einem offenen System, da die Feuchtigkeit auch über die neben dem Bohrloch vorhandene Oberfläche abgegeben wird. Daher ist diese Messmethode nur dann hinreichend genau, wenn das Bohrloch genügend tief ist.

Bei Betondecken wird in der Regel von der Oberseite her ein Loch bis in die Mitte der Decke gebohrt. Dies entspricht üblicherweise einer Tiefe von 10 cm, da Betondecken in der Regel eine Gesamtdicke von 20 bis 30 cm aufweisen.

Unterlagsböden werden normalerweise mit einer Gesamtdicke von 4 bis 8 cm eingebracht. Daher ist die Anwendung dieser Messmethode bei Unterlagsböden mit einem erheblich grösseren Fehler behaftet, weil nicht genügend tief gebohrt werden kann.

Die Bestimmung der Luftfeuchtigkeit in einem Bohrloch in einem Unterlagsboden oder in einer Betondecke hat ausserdem den Nachteil, dass das Messergebnis erst nach 5 bis 7 Tagen erhältlich ist und massgeblich von den Umgebungsbedingungen abhängt.

Bis zum Datum der Hinterlegung der vorliegenden Patentanmeldung existiert keine Möglichkeit, die Messergebnisse der beiden oben genannten Methoden miteinander zu korrelieren.

Es ist ein Ziel der vorliegenden Erfindung, die oben genannten Nachteile zu überwinden.

Es ist ein weiteres Ziel der vorliegenden Erfindung, eine Vorrichtung zur Bestimmung der korrespondierenden Luftfeuchtigkeit einer Materialprobe und zur Bestimmung des Feuchtigkeitsgehaltes dieser gleichen Materialprobe in einem geschlossenen System zur Verfügung zu stellen.

Mit der erfindungsgemässen Vorrichtung werden diese Ziele erreicht.

Die erfindungsgemässe Vorrichtung zur Bestimmung der korrespondierenden Luftfeuchtigkeit einer Materialprobe und zur Bestimmung des Feuchtigkeitsgehaltes dieser gleichen Materialprobe in einem geschlossenen System,
ist dadurch gekennzeichnet, dass diese Vorrichtung nebst einer Luftfeuchte-/Lufttemperaturmessvorrichtung 1 und einer Druckmessvorrichtung 2
- eine verschliessbare Druckflasche 3,
- einen ersten Adapter 4, welcher mit der genannten Druckflasche 3 druckdicht verschliessbar ist und eine durchgehende Öffnung 5 aufweist, welche staubdicht verschliessbar ist, und zur Aufnahme der Luftfeuchte-/Lufttemperaturmessvorrichtung 1 ausgebildet ist, und
- einen zweiten Adapter 6, welcher mit der genannten Druckflasche 3 druckdicht verschliessbar ist und eine durchgehende Öffnung 7 aufweist und zur Aufnahme der Druckmessvorrichtung 2 ausgebildet ist,
   umfasst.

Bevorzugte Ausführungsformen der vorliegenden Erfindung sind in den abhängigen Ansprüchen definiert.

Im folgenden Teil werden mögliche Ausführungsformen beschrieben. Dabei wird Bezug auf die Figuren genommen.

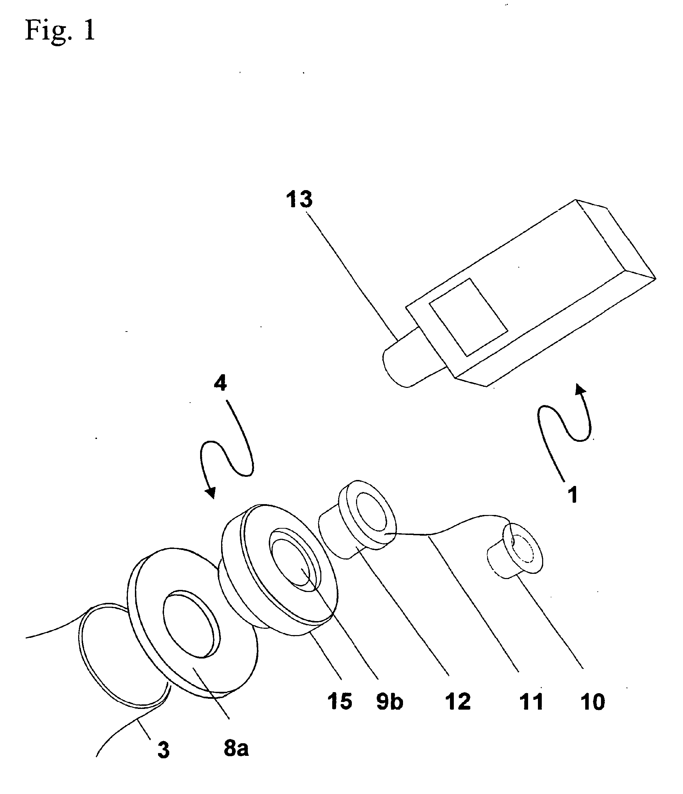

Figur 1 zeigt rein schematisch eine Explosionsdarstellung einer möglichen Ausführungsform des ersten Adapters 4 zur Bestimmung der korrespondierenden Luftfeuchtigkeit einer Materialprobe. Dabei sind die beiden Bügelverschlüsse an der Druckflasche 3 nicht eingezeichnet.

Figur 2A zeigt rein schematisch einen möglichen ersten Adapter 4 von links unten, ausgehend vom ersten zylindrischen Teil 14.

Figur 2B zeigt rein schematisch einen möglichen ersten Adapter 4 von links unten, ausgehend vom zweiten zylindrischen Teil 15.

Figur 2C zeigt rein schematisch einen Querschnitt durch einen möglichen ersten Adapter 4.

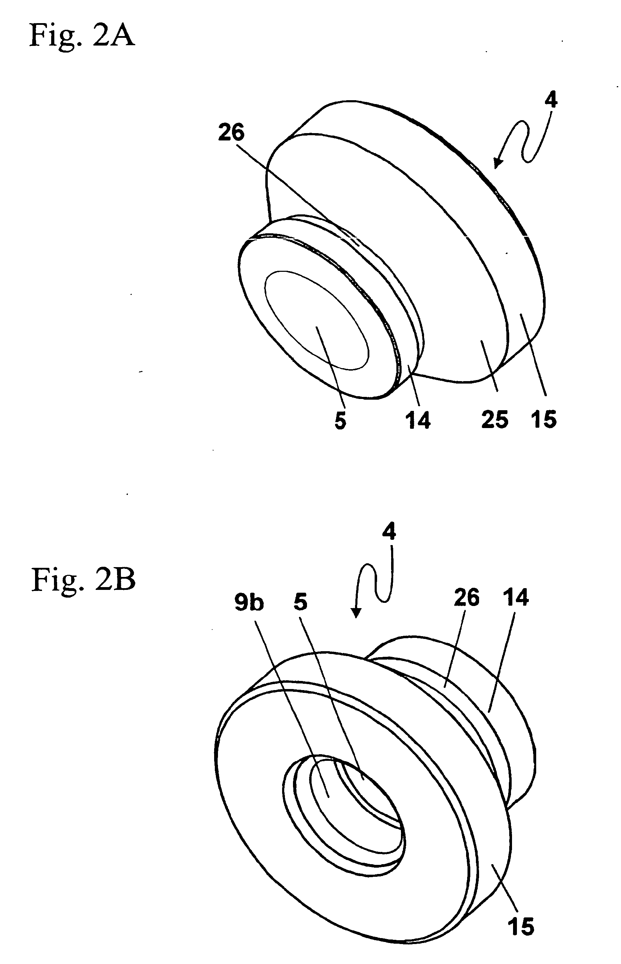

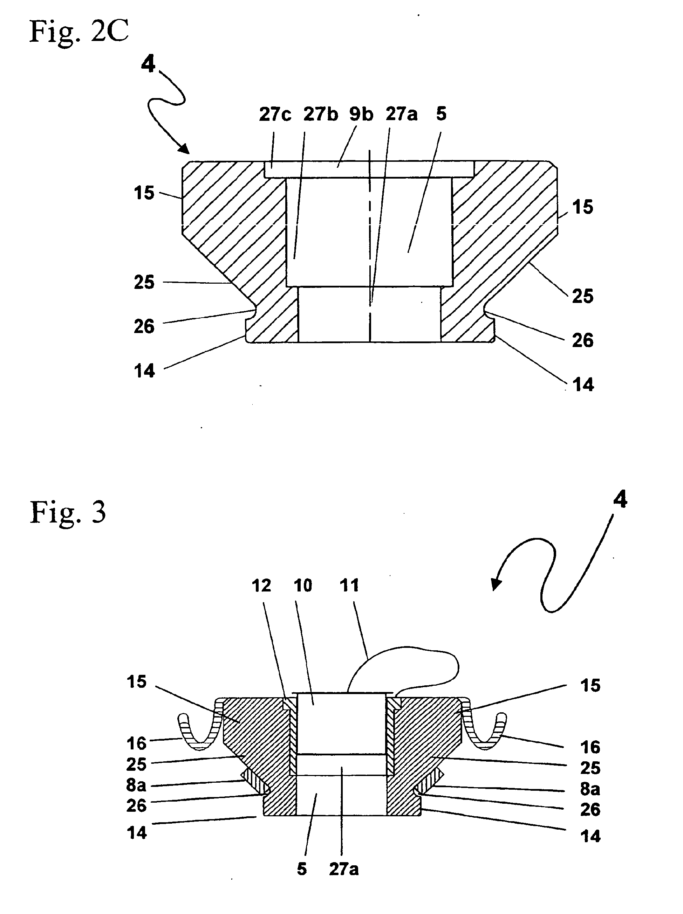

In den Figuren 2A, 2B, 2C sind die beiden Schliesshaken, das Gleitlager 12, die Schutzkappe 10 und die Nylonschnur 11 nicht eingezeichnet.

Figur 3 zeigt rein schematisch einen Querschnitt durch einen möglichen ersten Adapter 4 in fertig montierter Form.

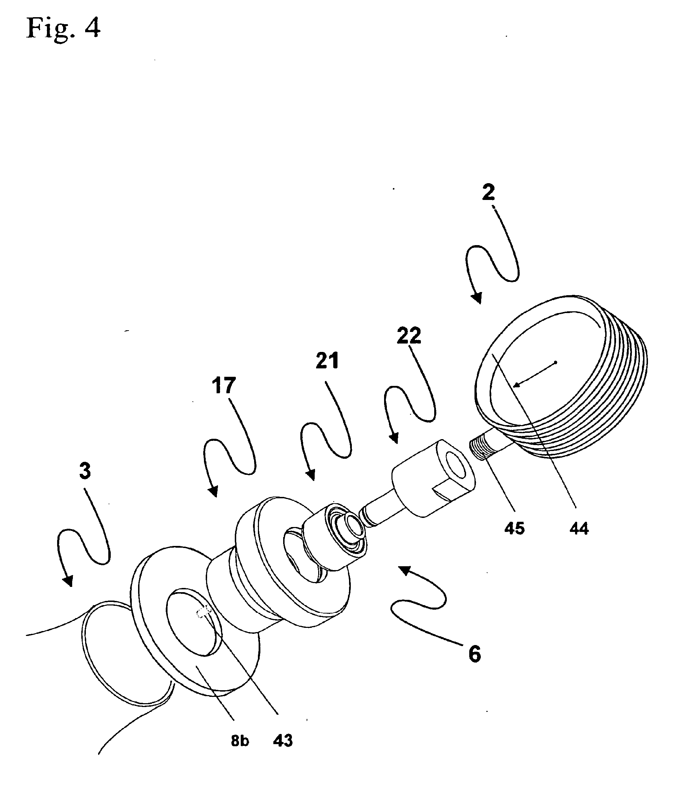

Figur 4 zeigt rein schematisch eine Explosionsdarstellung einer möglichen Ausführungsform eines zweiten Adapters 6. Dabei sind die beiden Bügelverschlüsse an der Druckflasche 3 nicht eingezeichnet.

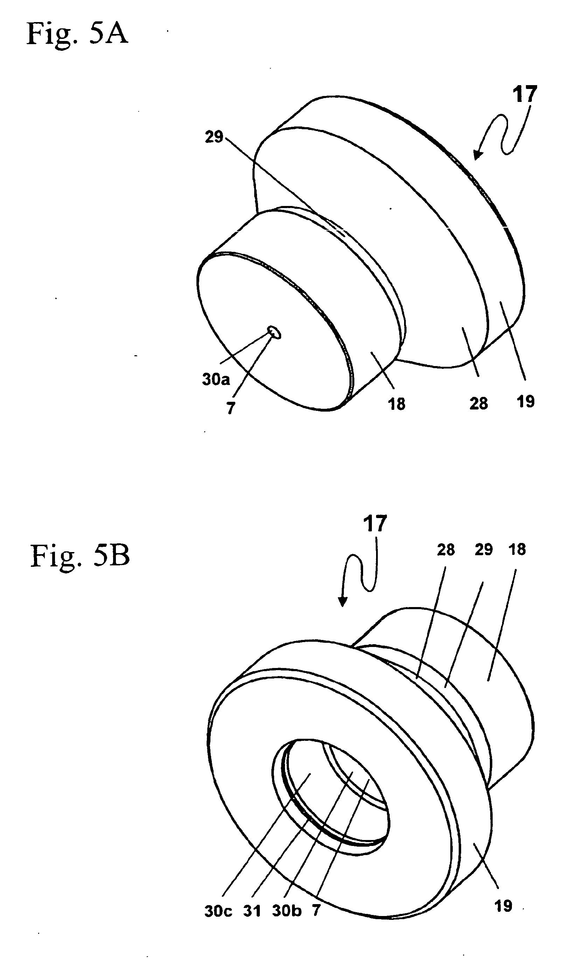

Figur 5A zeigt rein schematisch ein mögliches erstes Verbindungsteil 17 von links unten, ausgehend vom ersten zylindrischen Teil 18 des zweiten Adapters 6.

Figur 5B zeigt rein schematisch ein mögliches erstes Verbindungsteil 17 von links unten, ausgehend vom zweiten zylindrischen Teil 19 des zweiten Adapters 6.

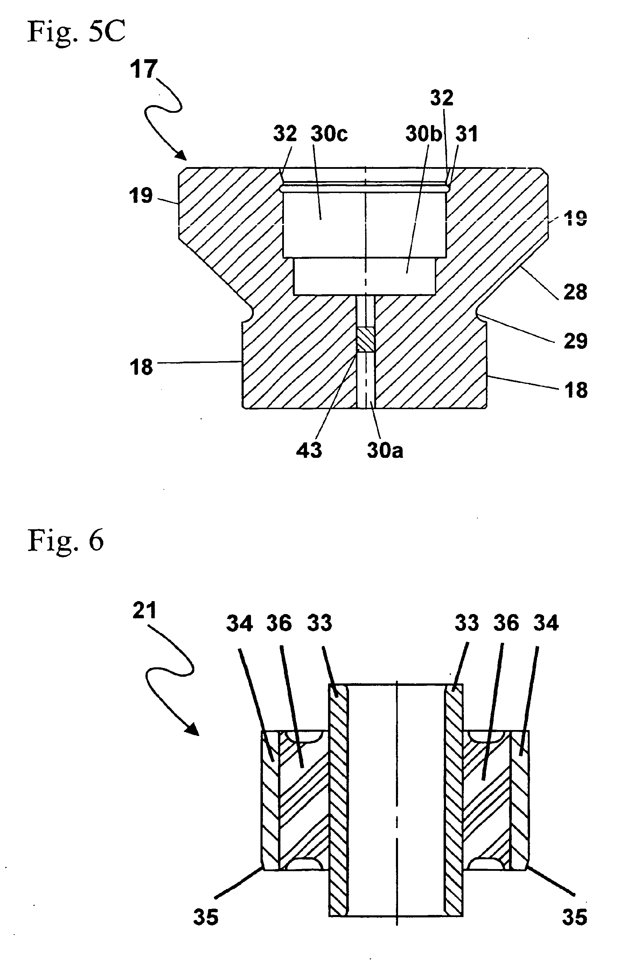

Figur 5C zeigt rein schematisch einen Querschnitt durch ein mögliches erstes Verbindungsteil 17 des zweiten Adapters 6.

In den Figuren 5A, 5B, 5C sind die beiden Schliesshaken 20 nicht eingezeichnet.

Figur 6 zeigt rein schematisch einen Querschnitt durch eine mögliche Ausführungsform des Dämpfungsteiles 21 des zweiten Adapters 6.

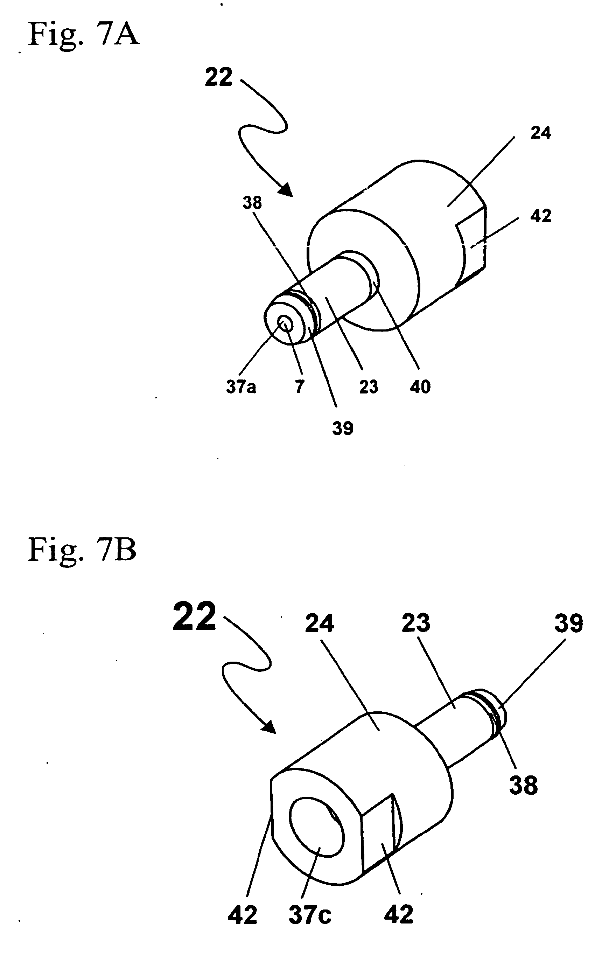

Figur 7A zeigt rein schematisch ein mögliches zweites Verbindungsteil 22 von links unten, ausgehend vom ersten zylindrischen Teil 23 des zweiten Adapters 6.

Figur 7B zeigt rein schematisch ein mögliches zweites Verbindungsteil 22 von links unten, ausgehend vom zweiten zylindrischen Teil 24 des zweiten Adapters 6.

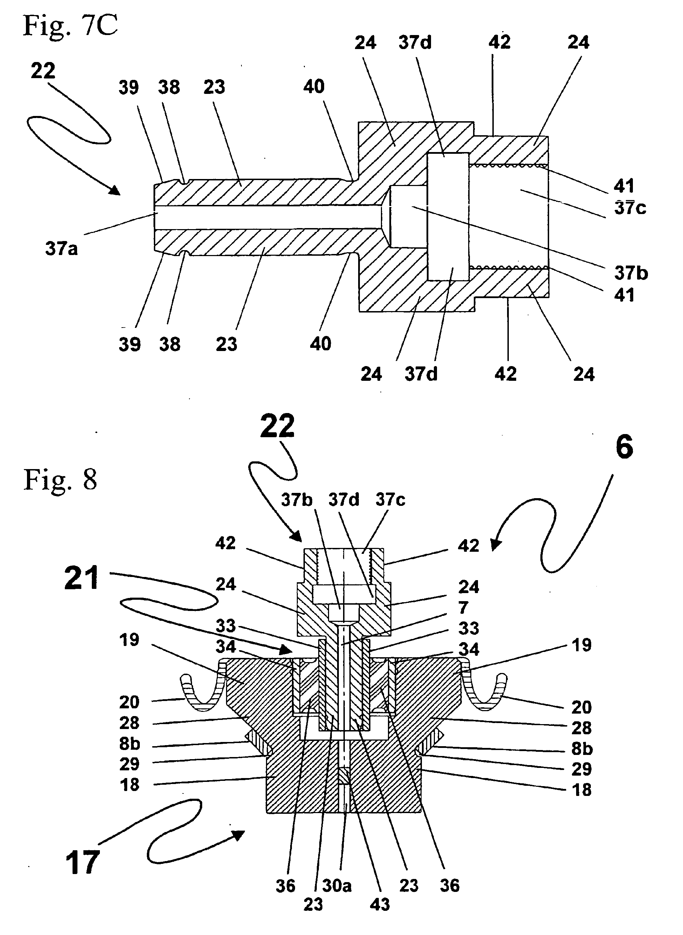

Figur 7C zeigt rein schematisch einen Querschnitt durch ein mögliches zweites Verbindungsteil 22 des zweiten Adapters 6.

Figur 8 zeigt rein schematisch einen Querschnitt durch einen möglichen zweiten Adapter 6 in montierter Form.

Gemäss einer möglichen ersten Ausführungsform der vorliegenden Erfindung wird als Druckflasche 3 eine konisch zugespitzte Flasche aus Edelstahl verwendet. Der Bodendurchmesser beträgt 90 mm und die Flaschenhöhe 165 mm. Der Durchmesser der Flaschenöffnung beträgt 43.2 mm. Seitlich sind auf der Höhe der Flaschenöffnung zwei gegenüberliegende Bügelverschlüsse angeschweisst. Der Flascheninhalt beträgt etwa 760 ml. Der Boden der Flasche ist abgerundet und weist einen Radius von 16 mm auf.

Auf der Druckflasche 3 kann ein Oberflächenthermometer aufgeklebt sein, welches einen Hinweis auf die Flaschentemperatur gibt.

Als Folge einer Volumeneichung kann der Boden der Druckflasche 3 nach aussen oder nach innen gewölbt sein.

Der erste Adapter 4 ist aus Edelstahl hergestellt und weist zwei gerade zylindrische Teile 14,15 auf, die miteinander über einen geraden Kegelstumpf 25 konzentrisch verbunden sind.

Der Durchmesser des ersten zylindrischen Teils 14 beträgt 33 mm und die Höhe 2.5 mm. Der Durchmesser des zweiten zylindrischen Teils 15 beträgt 50 mm und die Höhe 10 mm. Die beiden Durchmesser des genannten geraden Kegelstumpfes 25 sind mit den beiden zylindrischen Teilen 14,15 bündig. Der Kegelstumpf 25 hat eine Höhe von 8.8 mm. Zwischen dem ersten zylindrischen Teil 14 und dem Kegelstumpf 25 ist auf dem ersten zylindrischen Teil 14 eine Aussenkerbe 26 mit Radius von 1.5 mm angebracht.

Im ersten Adapter 4 sind drei zentrische axiale Bohrungen 27a,27b,27c vorhanden.

Ausgehend vom ersten zylindrischen Teil 14 ist eine durchgehende axiale Bohrung 27a mit einem Durchmesser von 12 mm vorhanden.

Ausgehend vom zweiten zylindrischen Teil 15 sind zwei axiale Bohrungen 27b,27c vorhanden. Die erste Bohrung 27b hat einen Durchmesser von 16 mm und eine Tiefe von 19 mm. Die zweite Bohrung 27c hat einen Durchmesser von 21 mm und eine Tiefe von 2 mm.

Am ersten Adapter 4 sind ausgehend vom zweiten zylindrischen Teil 15 seitlich zwei gegenüberliegende Schliesshaken 16 angebracht, die auf die Bügelverschlüsse der Druckflasche 3 abgestimmt sind.

Der Dichtring 8a ist aus Ethylen-Propylen-Dien-Kautschuk, EPDM, hergestellt und ist 4 mm dick und hat einen Aussenradius von 48 mm und einen Innenradius von 28 mm. Der Dichtring 8a hat eine Shore A Härte von 50 und wird auf die Aussenkerbe 26 des ersten Adapters 4 platziert.

Ausgehend vom zweiten zylindrischen Teil 15 ist in die beiden axialen Bohrungen 27b,27c ein passendes zylindrisches Gleitlager 12 gepresst.

Dieses Gleitlager 12 ist vorzugsweise aus Nylon-66 hergestellt. Geeignete Gleitlager 12 sind bei der Firma Johan Pützfeld Industrie en Handelscompagnie B.V. in NL-1014 AJ Amsterdam/Niederlande erhältlich, beispielsweise das Produkt mit der Artikelnummer: 008 1200 000 02.

Zwischen dem Gleitlager 12 und dem zweiten zylindrischen Teil 15 des ersten Adapters 4 kann eine Nylonschnur oder ein Metalldraht 11 mit geeigneter Länge, beispielsweise 5 cm, eingepresst sein. Auf der anderen Seite der Nylonschnur oder des Metalldrahtes 11 kann die Schutzkappe oder der Schutzstopfen 10 fixiert sein. Diese Fixation kann beispielsweise mittels eines Heissleimes erfolgen.

Diese Schutzkappe oder dieser Schutzstopfen 10 ist vorzugsweise aus Polyethylen (LDPE) hergestellt. Geeignete Schutzkappen sind bei der Firma Johan Pützfeld Industrie en Handelscompagnie B.V. in NL-1014 AJ Amsterdam/Niederlande erhältlich, beispielsweise das Produkt mit der Artikelnummer: 004 0105 005 03.

Als Luftfeuchte-/Lufttemperaturmessvorrichtung 1 kann ein Gerät der Firma Trotec Handelsgesellschaft für Industriegüter in D-52525 Heinsberg/Deutschland verwendet werden, beispielsweise die Handmessgeräte T200 oder T250 oder der Sensor TS200 SDI zum Anzeigegerät T2000.

Der zweite Adapter 6 umfasst zwei Verbindungsteile 17,22 und ein Dämpfungsteil 21.

Das erste Verbindungsteil 17 ist aus Edelstahl hergestellt und weist zwei gerade zylindrische Teile 18,19 auf, die miteinander über einen geraden Kegelstumpf 28 konzentrisch verbunden sind.

Der Durchmesser des ersten zylindrischen Teils 18 beträgt 33 mm und die Höhe 15.2 mm. Der Durchmesser des zweiten zylindrischen Teils 19 beträgt 50 mm und die Höhe 10 mm. Die beiden Durchmesser des genannten geraden Kegelstumpfes 28 sind mit den beiden zylindrischen Teilen 18,19 bündig. Der Kegelstumpf 28 hat eine Höhe von 8.8 mm. Zwischen dem ersten zylindrischen Teil 18 und dem Kegelstumpf 28 ist auf dem ersten zylindrischen Teil 18 eine Aussenkerbe 29 mit Radius von 1.5 mm angebracht.

Im ersten Verbindungsteil 17 sind mehrere axiale Bohrungen 30a,30b,30c vorhanden.

Ausgehend vom ersten zylindrischen Teil 18 ist eine axiale Bohrung 30a mit einem Durchmesser von 2.5 mm und einer Tiefe von 16 mm vorhanden.

Ausgehend vom zweiten zylindrischen Teil 19 sind zwei axiale Bohrungen 30b,30c vorhanden, nämlich eine erste Bohrung 30b mit einem Durchmesser von 19 mm und einer Tiefe von 18 mm und eine weibliche Passung in der Form einer Bohrung 30c mit einem Durchmesser von 22 mm (H8/u10) und einer Tiefe von 12.5 mm.

Ausgehend vom zweiten zylindrischen Teil 19 ist in der weiblichen Passung 30c in einer Tiefe von 3 mm eine Innenkerbe 31 mit einem Radius von 0.5 mm ausgebildet. Die Oberfläche der weiblichen Passung 30c hat eine Rauheit von N7. Ausgehend vom zweiten zylindrischen Teil 19 ist in der weiblichen Passung 30c eine Innenfacette 32 ausgebildet, welche eine Länge von 2 mm und einen Winkel von 15° hat.

Am ersten Verbindungsteil 17 sind ausgehend vom zweiten zylindrischen Teil 19 seitlich zwei gegenüberliegende Schliesshaken 20 angebracht, die auf die Bügelverschlüsse der Druckflasche 3 abgestimmt sind.

Das Dämpfungsteil 21 ist als Stahl-Gummi Buchse ausgebildet. Es umfasst eine Innenwandung 33 in der Form eines Innenrohres aus Stahl, welches einen Innendurchmesser von 8 mm, eine Wandstärke von 1.6 mm und eine Länge von 20 mm hat, und eine Aussenwandung 34 in der Form eines Aussenrohres aus Stahl, welches einen Aussendurchmesser von 22 mm, eine Wandstärke von 1.6 mm und eine Länge von 12 mm hat. Das Aussenrohr 34 weist auf einer Seite eine Aussenfacette 35 auf, welche eine Länge von 2 mm und einen Winkel von 15° hat und auf die entsprechende Innenfacette 32 des ersten Verbindungsteils 17 abgestimmt ist. Das Innenrohr 33 und das Aussenrohr 34 sind konzentrisch und gemittet angeordnet und mit einer elastischen Füllung 36 aus Natur-Kautschuk der Shore A Härte von 60 miteinander dicht verbunden, und zwar mittels einer Vulkanisation des Gummis mit dem Stahl. Eine solche Stahl-Gummi Buchse 21 ist bei der Firma "Schwab Schwingungstechnik AG" in CH-8153 Adliswil erhältlich.

Das zweite Verbindungsteil 22 ist aus Edelstahl hergestellt und weist zwei konzentrisch angeordnete gerade zylindrische Teile 23,24 auf.

Das erste zylindrische Teil 23 hat einen Aussendurchmesser von 8 mm (z8) und eine Länge von 20.5 mm. Das zweite zylindrische Teil 24 hat einen Aussendurchmesser von 20 mm und eine Länge von 20 mm.

Im zweiten Verbindungsteil 22 sind mehrere axiale Bohrungen 37a,37b,37c,37d vorhanden.

Ausgehend vom ersten zylindrischen Teil 23 ist eine axiale Bohrung 37a mit einem Durchmesser von 2.5 mm und einer Tiefe von 24 mm vorhanden. Die Aussenoberfläche des ersten zylindrischen Teils 23 hat eine Rauheit von N7. Ausgehend vom ersten zylindrischen Teil 23 ist im Abstand von 3 mm eine erste Aussenkerbe 38 mit einem Radius von 0.5 mm ausgebildet. Ausgehend vom ersten zylindrischen Teil 23 ist eine Aussenfacette 39 mit einer Länge von 2 mm und einem Winkel von 15° ausgebildet und auf das entsprechende Innenrohr 33 des Dämpfungsteiles 21 abgestimmt.

Zwischen dem ersten zylindrischen Teil 23 und dem zweiten zylindrischen Teil 24 ist auf dem ersten zylindrischen Teil 23 eine 2 mm lange zweite Aussenkerbe 40 mit einer Tiefe von 0.4 mm angebracht.

Ausgehend vom zweiten zylindrischen Teil 24 sind mehrere axiale Bohrungen 37b,37c,37d vorhanden, nämlich eine erste Bohrung 37b mit einem Durchmesser von 6.6 mm und einer Tiefe von 16 mm, eine zweite Bohrung 37c mit einem Durchmesser von 10.2 mm und einer Tiefe von 12.2 mm und eine dritte Bohrung 41 in der Form einer Gewindebohrung G ¼" und einer Tiefe von 10 mm. In einer Tiefe von 8 mm bis 12.2 mm ist eine vierte Bohrung 37d in der Form eines Hinterstichs mit einem Durchmesser von 13.5 mm ausgebildet.

Ausgehend vom zweiten zylindrischen Teil sind an der Aussenseite zwei parallele gegenüberliegende Fräsungen 42 vorhanden, wobei die beiden parallelen Flächen eine Breite von 10.5 mm und eine Tiefe von 7.5 mm aufweisen. Diese Fräsungen 42 dienen dem Festhalten der montierten zweiten Adapters 6 mit einem Schraubenschlüssel der Schlüsselweite 17 bei der späteren Montage einer Druckmessvorrichtung 2.

In die durchgehende Öffnung 7 im zweiten Adapter 6 kann ein Drosselungsteil 43 eingepresst sein. Dieses Drosselungsteil 43 kann die Form eines Zylinders mit dem Durchmesser 3 mm und der Länge 5 mm haben und aus gesintertem High Density Polyethylen, HDPE, hergestellt sein.

Der Dichtring 8b ist aus Ethylen-Propylen-Dien-Kautschuk, EPDM, hergestellt und ist 4 mm dick und hat einen Aussenradius von 48 mm und einen Innenradius von 28 mm. Der Dichtring 8b hat eine Shore A Härte von 50 und wird auf die Aussenkerbe 29 des zweiten Adapters 6 platziert.

Für die Montage des zweiten Adapters 6 wird wie folgt vorgegangen:

Die Innenkerbe 31 des ersten Verbindungsteils 17 wird mit einem abdichtenden Klebstoff, beispielsweise Loctite 577, gefüllt. Anschliessend wird das Dämpfungsteil 21 mit der Aussenfacette 35 voran auf die weibliche Passung 30c des ersten Verbindungsteils 17 aufgesetzt und ausgehend von der Oberfläche des zweiten zylindrischen Teils 19 bündig eingepresst. Dabei muss die Presskraft auf das Aussenrohr 34 des Dämpfungsteils 21 wirken.

Die Aussenkerbe 38 des zweiten Verbindungsteils 22 wird mit einem abdichtenden Klebstoff, beispielsweise Loctite 577, gefüllt. Anschliessend wird das zweite Verbindungsteil 22 mit der Aussenfacette 39 voran auf das Innenrohr 33 des Dämpfungsteils 21 aufgesetzt und wird bis zum Anschlag eingepresst.

Das Drosselungsteil 43 wird ausgehend vom ersten zylindrischen Teil 18 des ersten Verbindungsteils 17 in die axiale Bohrung 30a etwa mittig eingepresst.

Als Druckmessvorrichtung 2 wird ein Rohrfeder-Manometer mit einem Durchmesser 80 mm, mit einem Aussengewinde G ¼" sowie einer passenden Dichtung verwendet. Der Skalenendwert dieses Manometers 2 beträgt 2.5 bar und die Genauigkeitsklasse ist 1.0. Dieses mechanische Manometer 2 ist mit einer passenden Gummimanschette 44 mit Dämpfungsrippen ummantelt.

Das Aussengewinde 45 des Manometers 2 wird in das Gewinde 41 des zweiten Verbindungsteils 22 dicht und fest eingeschraubt.

Gemäss einer möglichen zweiten Ausführungsform kann im ersten Adapter 4 das zweite zylindrische Teil 15 einen Durchmesser von 60 mm und eine Höhe von 10 mm aufweisen. Die beiden Durchmesser des genannten geraden Kegelstumpfes 25 können mit den beiden zylindrischen Teilen 14,15 bündig sein. Es ist bevorzugt, dass der Durchmesser des Kegelstumpfes 25 auf der Seite, welche zum zweiten zylindrischen Teil 15 gerichtet ist, 50 mm beträgt.

Gemäss dieser Ausführungsform ist im zweiten zylindrischen Teil 15 auf der nach Aussen gerichteten Seite eine konzentrische Kerbe mit einem Innendurchmesser von 44 mm, einem Aussendurchmesser von 55 mm und einer Tiefe von etwa 3 mm vorhanden.

Diese Kerbe dient als Verbindung zu einer Druckflasche 3 mit beispielsweise Hakenverschlüssen.

Zur Bestimmung der korrespondierenden Luftfeuchtigkeit einer Materialprobe, beispielsweise aus einem Unterlagsboden stammend, wird wie folgt vorgegangen:

Für die einzusetzende Probenmenge muss berücksichtigt werden, dass im Anschluss an die Bestimmung der korrespondierenden Luftfeuchtigkeit einer Materialprobe auch der Feuchtigkeitsgehalt dieser gleichen Materialprobe nach der Calciumcarbid-Methode bestimmt werden soll.

Daher wird je nach der zu erwartenden Feuchtigkeit eine bestimmte standardisierte Menge des Probenmaterials (beispielsweise 3 Gramm, 5 Gramm, 10 Gramm, 20 Gramm, 50 Gramm oder 100 Gramm) präzise abgewogen und in die Druckflasche 3 eingefüllt.

In die Druckflasche 3 werden ebenfalls drei Stahlkugeln mit einem Durchmesser 31/32" und eine Stahlkugel mit einem Durchmesser 14 mm eingefüllt.

Diese Stahlkugeln dienen in der Folge dazu, die Oberfläche des Probenmaterials in der Druckflasche 3 zu vergrössern, damit der Gleichgewichtszustand zwischen der Feuchtigkeit des Probenmaterials und der Luftfeuchtigkeit in der Druckflasche 3 möglichst schnell erreicht wird. Wenn mehr als vier solcher Kugeln eingesetzt werden, dann stellt sich der genannte Gleichgewichtzustand schneller ein.

Auf die Druckflasche 3 wird der erste Adapter 4 gemäss der oben beschriebenen ersten Ausführungsform aufgesetzt. Anschliessend wird die Druckflasche 3 mit Hilfe der Bügelverschlüsse und der Schliesshaken 16 druckdicht verschlossen, und die durchgehende Öffnung 5 im ersten Adapter 4 wird mit der Schutzkappe 10 staubdicht verschlossen.

Dieses geschlossene System wird von Hand mit einer Kadenz von etwa 250 Mal pro Minute während etwa zwei Minuten kräftig und senkrecht geschüttelt. Dabei wird das eingefüllte Probenmaterial durch die Stahlkugeln zerkleinert. Anschliessend an diesen Zerkleinerungsvorgang wird die Druckflasche 3 derart bewegt, dass sich das zerkleinerte Probenmaterial in der Druckflasche 3 mit der Innenluft gut durchmischt. Dies kann durch Drehen der horizontal gehaltenen Druckflasche 3 oder durch Kippbewegungen in Längsrichtung der Druckflasche 3 erfolgen.

Der genannte Gleichgewichtszustand zwischen der Feuchtigkeit des Probenmaterials und der Luftfeuchtigkeit in der Druckflasche 3 stellt sich in etwa 10 Minuten ein.

Anschliessend wird die Schutzkappe 10 entfernt und eine Luftfeuchte-/Lufttemperaturmessvorrichtung 1 wird in die durchgehende Öffnung 5 des ersten Adapters 4 geschoben. Nach etwa zwei Minuten hat sich zwischen dem Sensor der Luftfeuchte-/Lufttemperaturmessvorrichtung 1 und der Innenluft der Druckflasche 3 das Gleichgewicht eingestellt, und die Luftfeuchtigkeit und die Lufttemperatur können abgelesen werden.

Zur Bestimmung des Feuchtigkeitsgehaltes der gleichen Materialprobe werden nun der erste Adapter 4 und die Luftfeuchte-/Lufttemperaturmessvorrichtung 1 von der aufrecht stehenden Druckflasche 3 entfernt. Unverzüglich danach wird in die schräg gehaltene Druckflasche 3 vorsichtig Calciumcarbid gegeben, welches in einer Glasampulle abgepackt ist. Anschliessend wird sofort der zweite Adapter 6 aufgesetzt, und die Druckflasche 3 wird mit Hilfe der Bügelverschlüsse und der Schliesshaken 20 druckdicht verschlossen.

Die Messung des Feuchtigkeitsgehaltes der gleichen Materialprobe wird durch manuelles, intensives und senkrechtes Schütteln der Druckflasche 3 gestartet. Dabei bewirken die Stahlkugeln, dass einerseits die Glasampulle mit dem Calciumcarbid zerbrochen wird und andererseits das Probenmaterial und das freigesetzte Calciumcarbid vermischt und weiter zerkleinert werden. Das in der Druckflasche 3 resp. im Probenmaterial enthaltene Wasser reagiert mit dem Calciumcarbid und bildet unter anderem ein Gas, nämlich Acetylen. Der entstehende Druck wird mit dem Manometer 2 gemessen und bildet die Messgrösse zur Bestimmung des Feuchtigkeitsgehaltes der Materialprobe.

Die Reaktion ist abgeschlossen sobald der Druck nicht mehr ansteigt. Am Manometer 2 wird der Druck abgelesen. Der entstandene Druck steht in Korrelation zum Wassergehalt der Probenmenge. Die Feuchte des Probenmaterials wird einer entsprechenden Eichtabelle entnommen.

Nach Abschluss der Messung wird die Druckflasche 3 zum Entweichen des Überdruckes langsam geöffnet und trocken gereinigt.

Durch eine Gegenüberstellung des erhaltenen Messwertes der korrespondierenden Luftfeuchtigkeit einer Materialprobe und des erhaltenen Messwertes des Feuchtigkeitsgehaltes der gleichen Materialprobe kann eine Korrelation dieser beiden Messmethoden ermittelt werden. Dadurch wird es möglich, die Vorteile der beiden Messverfahren zu nutzen. Dies führt zu einer grösseren Sicherheit in der Beurteilung der Belegereife von Unterlagsböden, resp. Betondecken.

In der vorliegenden Erfindung werden folgende Bezugszeichen verwendet:
- 1: Luftfeuchte-/Lufttemperaturmessvorrichtung
- 2: Druckmessvorrichtung/Manometer
- 3: Druckflasche
- 4: erster Adapter
- 5: durchgehende Öffnung im ersten Adapter 4
- 6: zweiter Adapter
- 7: durchgehende Öffnung im zweiten Adapter 6
- 8a,8b: Dichtring
- 9a,9b: nach Aussen gerichtete Öffnung des ersten Adapters 4
- 10: Schutzkappe/Schutzstopfen
- 11: Nylonschnur/Metalldraht
- 12: Gleitlager
- 13: Schutzkappe
- 14: erstes zylindrisches Teil im ersten Adapter 4
- 15: zweites zylindrisches Teil im ersten Adapter 4
- 16: Schliesshaken am ersten Adapter 4
- 17: erstes Verbindungsteil im zweiten Adapter 6
- 18: erstes zylindrisches Teil im ersten Verbindungsteil 17
- 19: zweites zylindrisches Teil im ersten Verbindungsteil 17
- 20: Schliesshaken am zweiten Adapter 6
- 21: Dämpfungsteil
- 22: zweites Verbindungsteil im zweiten Adapter 6
- 23: erstes zylindrisches Teil im zweiten Verbindungsteil 22
- 24: zweites zylindrisches Teil im zweiten Verbindungsteil 22
- 25: gerader Kegelstumpf im ersten Adapter 4
- 26: Aussenkerbe des ersten Adapters 4
- 27a,27b,27c: zentrische axiale Bohrungen im ersten Adapter 4
- 27: gerader Kegelstumpf im zweiten Adapter 6
- 28: Aussenkerbe
- 30a,30b: axiale Bohrungen im zweiten Adapter 6
- 30c: axiale Bohrung/weibliche Passung im zweiten Adapter 6
- 31: Innenkerbe
- 32: Innenfacette
- 33: Innenwandung/Innenrohr
- 34: Aussenwandung/Aussenrohr
- 35: Aussenfacette
- 36: elastische Füllung
- 37a,37b,37c,37: d axiale Bohrungen im zweiten Verbindungsteil 22
- 38: Aussenkerbe
- 39: Aussenfacette
- 40: Aussenkerbe
- 41: Gewindebohrung
- 42: Fräsungen
- 43: Drosselungsteil
- 44: Gummimanschette
- 45: Aussengewinde des Manometers 2.

## Patentansprüche

1. Vorrichtung zur Bestimmung der korrespondierenden Luftfeuchtigkeit einer Materialprobe und zur Bestimmung des Feuchtigkeitsgehaltes dieser gleichen Materialprobe in einem geschlossenen System,
**dadurch gekennzeichnet, dass** diese Vorrichtung nebst einer Luftfeuchte-/Lufttemperaturmessvorrichtung (1) und einer Druckmessvorrichtung (2)
- eine verschliessbare Druckflasche (3),
- einen ersten Adapter (4), welcher mit der genannten Druckflasche (3) druckdicht verschliessbar ist und eine durchgehende Öffnung (5) aufweist, welche staubdicht verschliessbar ist, und zur Aufnahme der Luftfeuchte-/Lufttemperaturmessvorrichtung (1) ausgebildet ist, und
- einen zweiten Adapter (6), welcher mit der genannten Druckflasche (3) druckdicht verschliessbar ist und eine durchgehende Öffnung (7) aufweist und zur Aufnahme der Druckmessvorrichtung (2) ausgebildet ist,
umfasst.

2. Vorrichtung nach Anspruch 1, **dadurch gekennzeichnet, dass** die Druckflasche (3) mit dem ersten Adapter (4) und mit dem zweiten Adapter (6) mittels Verschraubung oder mittels Bajonett-, Haken- oder Bügelverschlüssen verbindbar ist, insbesondere mittels Haken- oder Bügelverschlüssen, wobei die Verbindung zum ersten Adapter (4) und zum zweiten Adapter (6) vorzugsweise identisch ist.

3. Vorrichtung nach einem der Ansprüche 1 bis 2, **dadurch gekennzeichnet, dass** sich zwischen der Druckflasche (3) und dem ersten Adapter (4) und zwischen der Druckflasche (3) und dem zweiten Adapter (6) ein gegenüber den berührenden Medien inerter und passender Dichtring (8a,8b) befindet.

4. Vorrichtung nach einem der Ansprüche 1 bis 3, **dadurch gekennzeichnet, dass** die nach Aussen gerichtete Öffnung (9a) des ersten Adapters (4) mit einer Schutzkappe oder mit einem Schutzstopfen (10) luftdicht verschliessbar ist, und wobei diese Schutzkappe oder dieser Schutzstopfen (10) vorzugsweise mit dem ersten Adapter (4) beweglich verbunden ist, beispielsweise mit einer Nylonschnur oder mit einem Metalldraht (11).

5. Vorrichtung nach einem der Ansprüche 1 bis 4, **dadurch gekennzeichnet, dass** sich in der nach Aussen gerichteten Öffnung (9b) des ersten Adapters (4) ein hineingepresstes, zylindrisches Gleitlager (12), vorzugsweise aus Nylon-66, befindet, dessen Innendurchmesser auf den Aussendurchmesser der Schutzkappe (13) des Sensors der Luftfeuchte-/Lufttemperaturmessvorrichtung (1) abgestimmt ist.

6. Vorrichtung nach einem der Ansprüche 1 bis 5, **dadurch gekennzeichnet, dass** der Sensor der Luftfeuchte-/Lufttemperaturmessvorrichtung (1) in den Innenraum der Druckflasche (3) hineinragt und der Kontakt der Innenluft mit dem Sensor nicht durch den Adapter (4) behindert ist.

7. Vorrichtung nach einem der Ansprüche 1 bis 6, **dadurch gekennzeichnet, dass** die Verbindung zwischen dem zweiten Adapter (6) und der Druckmessvorrichtung (2) eine Press-Passung, eine Verschraubung, eine Verlötung oder eine Verklebung ist, einschliesslich die Kombinationen von Press-Passung mit entweder Verschweissung oder mit Verlötung oder mit Verklebung, oder die Kombinationen von Verschraubung mit entweder Verschweissung oder mit Verlötung oder mit Verklebung.

## Claims

1. Device for determining the corresponding humidity of a material sample and for determining the moisture content of this same material sample in a closed system,
**characterised in that**, this device, in addition to a humidity / air temperature measuring device (1) and a pressure measuring device (2), contains
- a sealable pressure bottle (3),
- a first adapter (4), which is air-tight sealable with the above mentioned pressure bottle (3) and has a through opening (5), which is dust-proof sealable, and is constructed to take the humidity / air temperature measuring device (1), and
- a second adapter (6), which is air-tight sealable with the above mentioned pressure bottle (3)and has a through opening (7) and is constructed to take the pressure measuring device (2).

2. Device according to claim 1, **characterised in that** a pressure bottle (3) can be connected with the first adapter (4) and the second adapter (6) by means of a screw joint or by means of bayonet, hook or clip fasteners, in particular by means of hook or clip fasteners, whereby the connection to the first adapter (4) and to the second adapter (6) is preferably identical.

3. Device according to either claim 1 or 2, **characterised in that** between the pressure bottle (3) and the first adapter (4) and between the pressure bottle (3) and the second adapter (6) a suitable sealing ring(8a,8b) is located, which is inert compared with the medium it comes into contact with.

4. Device according to any one of the claims 1 to 3, **characterised in that** the outward pointing opening (9a) of the first adapters (4) is air-tight sealable with a protective cap or protective stopper (10), and whereby this protective cap or this protective stopper (10) is preferably movably connected with the first adapter (4), for example with a nylon cord or with a metal wire (11).

5. Device according to any one of the claims 1 to 4, **characterised in that** in the outward pointing opening (9b) of the first adapter (4) an inwards pressed, cylindrical slide bearing (12) is located, preferably of Nylon 66, whose internal diameter is determined by the external diameter of the protective cap (13) of the sensor of the humidity / air temperature measuring device (1).

6. Device according to any one of the claims 1 to 5, **characterised in that** the sensor of the humidity / air temperature measuring device (1) projects into the inside of the pressure bottle (3) and the contact of the inside air with the sensor is not impaired by the adapter (4).

7. Device according to any one of the claims 1 to 6, **characterised in that** the connection between the second adapter (6) and the pressure measuring device (2) is a press-fit, a screw joint, soldering or adhesion, including the combination of press-fit with either welding or with soldering or with adhesion, or the combination of screw joint with either welding or with soldering or with adhesion.

## Revendications

1. Dispositif pour déterminer l'humidité de l'air correspondante d'un échantillon de matériau et pour déterminer la teneur en humidité de ce même échantillon de matériau dans un système fermé, **caractérisé en ce que** ce dispositif comprend, outre un dispositif (1) de mesure de l'humidité et de la température de l'air et un dispositif manométrique (2),
- une bouteille souple (3) pouvant être obturée,
- un premier adaptateur (4), qui peut être obturé d'une manière étanche à la pression avec ladite bouteille souple (3) et possède une ouverture traversante (5), qui peut être obturée d'une manière étanche aux poussières, et qui est configuré pour recevoir le dispositif (1) de mesure de l'humidité et de la température de l'air, et
- un deuxième adaptateur (6), qui peut être obturé d'une manière étanche à la pression avec ladite bouteille souple (3) et possède une ouverture traversante (7), et qui est configuré pour recevoir le dispositif manométrique (2).

2. Dispositif selon la revendication 1, **caractérisé en ce que** la bouteille souple (3) peut être raccordée au premier adaptateur (4) et au deuxième adaptateur (6) à l'aide d'un vissage, ou à l'aide de bouchons à baïonnette, à crochets ou à étrier, en particulier des bouchons à crochets ou à étrier, le raccordement au premier adaptateur (4) et celui au deuxième adaptateur (6) étant de préférence identiques.

3. Dispositif selon l'une des revendications 1 à 2, **caractérisé en ce qu'**un joint torique (8a, 8b), ajusté et inerte vis-à-vis des fluides avec lesquels il est en contact, se trouve entre la bouteille souple (3) et le premier adaptateur (4) et l'autre entre la bouteille souple (3) et le deuxième adaptateur (6).

4. Dispositif selon l'une des revendications 1 à 3, **caractérisé en ce que** l'ouverture (9a) du premier adaptateur (4), dirigée vers l'extérieur, peut être obturée d'une manière étanche à l'air avec un capot de protection ou avec un bouchon de protection (10), ce capot de protection ou ce bouchon de protection (10) étant de préférence raccordé d'une manière mobile au premier adaptateur (4), par exemple avec un cordon de Nylon ou avec un fil métallique (11).

5. Dispositif selon l'une des revendications 1 à 4, **caractérisé en ce que**, dans l'ouverture (9b) du premier adaptateur (4), dirigée vers l'extérieur, on a un palier lisse cylindrique (12), qui y a été enfoncé, de préférence en Nylon-66, dont le diamètre intérieur est adapté au diamètre extérieur du capot de protection (13) du capteur du dispositif (1) de mesure de l'humidité et de la température de l'air.

6. Dispositif selon l'une des revendications 1 à 5, **caractérisé en ce que** le capteur du dispositif (1) de mesure de l'humidité et de la température de l'air pénètre dans l'espace intérieur de la bouteille souple (3), le contact de l'air intérieur avec le capteur n'étant pas gêné par l'adaptateur (4).

7. Dispositif selon l'une des revendications 1 à 6, **caractérisé en ce que** le raccordement entre le deuxième adaptateur (6) et le dispositif manométrique (2) est un ajustement serré, un vissage, un brasage ou un collage, y compris la combinaison d'un ajustement serré et d'un soudage, ou d'un brasage, ou d'un collage, ou encore la combinaison d'un vissage et d'un soudage, ou d'un brasage, ou d'un collage.
